# EUROPEAN PATENT SPECIFICATION

(11) **EP 1 658 105 B1**
(45) Date of publication and mention of the grant of the patent: **14.03.2018**
(21) Application number: 04782101.2
(22) Date of filing: 25.08.2004
(51) Int. Cl.: A61K 9/12, B65D 83/00

(54) **PHARMACEUTICAL METERED DOSE INHALER AND METHODS RELATING THERETO**
PHARMAZEUTISCHER DOSIERINHALATOR UND RELEVANTE VERFAHREN
AEROSOL-DOSEUR PHARMACEUTIQUE ET PROCEDES ASSOCIES

(30) Priority: 29.08.2003 US 499250 P
(43) Date of publication of application: 24.05.2006
(73) Proprietor: GLAXO GROUP LIMITED, Middlesex TW8 9GS (GB)
(72) Inventor: Miller, John F., GlaxoSmithKline, Research Triangle Park, NC 27709 (US); Sommerville, Mark L., GlaxoSmithKline, Research Triangle Park, NC 27709 (US); Schultz, Robert D., GlaxoSmithKline, Research Triangle Park, NC 27709 (US)
(74) Representative: Rice, Jason Neale
(86) International application number: PCT/US2004/027539
(87) International publication number: WO 2005/023330

(56) References cited:
- EP-A2- 0 101 157
- WO-A1-2004/110335
- US-A- 4 919 312
- US-A- 5 830 490
- US-A- 5 899 200
- US-A- 6 036 942

## Description

### Field of the Invention

The present invention relates to medical devices useful in the treatment of respiratory disorders.

### Background of the Invention

The use of aerosols to administer medicaments has been known for several decades. Such aerosols generally comprise one or more medicaments, one or more propellants and optionally one or more additives, for example a surfactant or a co-solvent, such as ethanol. Historically the most commonly used aerosol propellants for medicaments have been propellant 11 (CCl₃F), propellant 114 (CF₂ClCF₂Cl), propellant 12 (CCl₂F₂) or combinations of those. However release of those propellants into the atmosphere is now believed to contribute to the degradation of stratospheric ozone and there is thus a need to provide aerosol formulations for medicaments which employ so called "ozone-friendly" propellants.

Containers for aerosol formulations commonly comprise a vial body (e.g., can or canister) coupled to a valve. The valve comprises a valve stem through which the formulations are dispensed. Generally the valve includes one or more rubber valve seals intended to allow reciprocal movement of the valve stem which prevents leakage of propellant from the container. Metered dose inhalers comprise a valve which is designed to deliver a metered amount of an aerosol formulation to the recipient per actuation. Such a metering valve generally comprises a metering chamber which is of a pre-determined volume and which causes the dose per actuation to be an accurate, pre-determined amount.

The metering valve in a container is typically coupled to the canister with contact through a sealing gasket to prevent leakage of propellant and/or drug substance out of the container at the join. The gasket typically comprises an elastomeric material, for example low density polyethylene, chlorobutyl, acrylonitrile butadiene rubbers, butyl rubber, a polymer of ethylene propylene diene monomer (EPDM), neoprene or chloroprene. Such elastomeric materials may be carbon-black or mineral filled.

Valves for use in MDIs are available from various manufactures known in the aerosol industry; for example from Valois, France (e.g. DF10, DF30, DF60), Bespak plc, UK (e.g. BK300, BK356, BK357) or 3M-Neotechnic Limited, UK (e.g. SpraymiserTM). The metering valves are used in association with commercially available canisters, for example metal canisters, for example aluminium canisters, suitable for delivering pharmaceutical aerosol formulations.

MDIs incorporating a valve seal or a sealing gasket as described above generally perform adequately with CFC propellants, such as propellant 11 (CCl₃F), propellant 114 (CF₂ClCF₂Cl), propellant 12 (CCl₂F₂). However, as mentioned above, there is a requirement to substitute so-called ozone-friendly propellants for CFC propellants in aerosols. A class of propellants which are believed to have minimal ozone-depleting effects in comparison to conventional chlorofluorocarbons comprise fluorocarbons and hydrogen-containing chlorofluorocarbons. That class includes, but is not limited to hydrofluoroalkanes (HFAs), for example 1,1,1,2-tetrafluoroethane (HFA134a), 1,1,1,2,3,3,3-heptafluoro-n-propane (HFA 227) and mixtures thereof. However, various problems have arisen with pharmaceutical aerosol formulations prepared using HFA propellants, in particular with regard to the stability of the formulations.

Pharmaceutical aerosol formulations generally comprise a solution or a suspension. A mixture of a suspension and a small amount of dissolved medicament is also possible, but generally undesirable (as described below). Some solution formulations have the disadvantage that the drug substance contained therein is more susceptible to degradation than when in solid form. Furthermore, solution formulations may be associated with problems in controlling the size of the droplets which in turn affects the therapeutic profile. Suspension formulations are thus generally preferred.

To obtain regulatory approval, pharmaceutical aerosol formulation products must satisfy strict specifications. One parameter that must generally be satisfied, and for which a level is usually specified, is the fine particle mass (FPM). The FPM is a measure of the amount of drug that has the potential to reach the inner lungs (the small bronchioles and alveoli) based on the proportion of drug particles with a diameter within a certain range, usually less than 5 microns. The FPM of an actuation from an MDI is generally calculated on the basis of the sum of the amount of drug substance deposited on stages 3, 4 and 5 of an Andersen Cascade Impaction stack as determined by standard HPLC analysis. Potential side effects are minimised and a smaller amount of drug substance is wasted if the FPM constitutes as large as possible a percentage of the total mass of drug.

In suspension formulations, particle size of the emitted dose is generally controlled during manufacture by the size to which the solid medicament is reduced, usually by micronisation. During storage of some drug suspensions in an HFA, however, various changes have been found to take place which have the effect of reducing FPM. A drop in FPM means that the therapeutically effective amount of drug available to the patient is reduced. That is undesirable and may ultimately impact on the effectiveness of the medication. That problem is particularly acute when the dose due to be dispensed is low, which is the case for certain potent drugs such as long acting beta agonists, which are bronchodilators.

Various mechanisms have been proposed by which the reduction in FPM may be taking place: particle size growth may occur if the suspended drug has a sufficient solubility in propellant, a process known as Ostwald Ripening. Alternatively, or additionally, small particles may have the tendency to aggregate or adhere to parts of the inside of the MDI, for example the canister or valve. Small particles may also become absorbed into or adsorbed onto rubber components of the valve. As adherence and absorption processes are more prevalent amongst small particles, those processes may lead to a decrease in FPM as a fraction of the administered drug as well as a reduction in the total drug content (TDC) of the canister available to patient. It has further been found that the adherence and absorption processes may not only result in loss of available drug, but may also adversely affect the function of the device, resulting in the valve sticking or orifices becoming blocked.

It is essential that the prescribed dose of aerosol medication delivered from the MDI to the patient consistently meets the specifications claimed by the manufacturer and complies with the requirements of the FDA and other regulatory authorities. That is, every dose dispensed from the MDI must be the same within close tolerances. Therefore it is important that the formulation be substantially homogenous throughout the canister and the administered dose at the time of actuation of the metering valve and that it remains substantially the same even after storage.

Various approaches have been taken to address the problems mentioned above. One approach is the addition of one or more adjuvants to the drug suspension; for example adjuvants selected from alcohols, alkanes, dimethyl ether, surfactants (e.g. fluorinated or non-fluorinated surfactants, carboxylic acids, polyethoxylates, etc.) and even conventional chlorofluorocarbon propellants in small amounts (at levels intended to keep to a minimum potential ozone damage) have been shown to have some effect in mitigating the FPM problems. Such approaches have been disclosed, for example, in EP0372777, WO91/04011, WO91/11173, WO91/11495 and WO91/14422. WO92/00061 discloses non-fluorinated surfactants for use with fluorocarbon propellants. Fluorinated surfactants may be used to stabilise micronised drug suspensions in fluorocarbon propellants such as 1,1,1,2-tetrafluoroethane (P134a) or 1,1,1,2,3,3,3-heptafluoro-n-propane (P227), see for example US4,352,789, US5,126,123, US5,376,359, US application 09/580,008, WO91/11173, WO91/14422, WO92/00062 and WO96/09816.

In WO96/32345, WO96/32151, WO96/32150 and WO96/32099 there are disclosed aerosol canisters coated with one or more fluorocarbon polymers, optionally in combination with one or more non-fluorocarbon polymers, that reduce the deposition on the canister walls of drug particles of the pharmaceutical alternative propellant aerosol formulation contained therein.

In WO 03/049786 it is described that deposition of drug on an elastomeric seal, and several other problems associated with lubrication, flexibility and sealing ability of an elastomeric seal may be overcome by the addition of an organotitanium low friction barrier coating to the seal surface. A pre-treatment step in which the elastomeric seal is treated as follows is also disclosed therein: the elastomeric substrate is provided in a bath comprising an alcohol and an alkaline material at a bath temperature effective for treatment, ultrasonic energy is provided to the bath at a treatment effective frequency and power level for a time sufficient to treat the elastomeric substrate, the treated elastomeric substrate is rinsed with de-ionised water; and the treated and rinsed elastomeric substrate is dried. The pre-treatment step is said to permit superior adhesion and bonding of the organotitanium-based coating. In general, however, additional material coating steps add to the expense of manufacturing the final drug product and the presence of a coating may cause additional toxicity and safety tests to be necessary.

**T**he present invention is concerned with medical devices, such as metered dose inhalers, that may provide improved stability of pharmaceutical formulations contained therein.

### Summary of the Invention

Applicants have surprisingly discovered that when the neck (or cap) seal of a metered dose inhaler (MDI) comprises a different elastomeric material than the material used for a stem seal in the metering valve of the (MDI), a pharmaceutical formulation contained in the MDI can exhibit an improved stability (e.g., a decreased drop in FPM after storage) compared to an MDI in which the cap seal and the stem seal comprise the same elastomeric material.

According to the present invention, there is provided a medicament dispenser according to claim 1 appended hereto. The other appended claims set out preferred features or embodiments of the invention.

According to embodiments of the present invention, a metered dose inhaler includes a medicament dispenser according to the present invention, and an actuator engaging the medicament dispenser and configured to dispense the pharmaceutical composition from the medicament dispenser.

According to embodiments of the present invention, a packaging material forming an enclosed volume contains the metered dose inhaler.

### Brief Description of the Drawings

**Figure 1** illustrates a sealed container according to embodiments of the present invention;
**Figure 2** illustrates a sectional view taken along the line I-I of a portion of the sealed container illustrated in **Figure 1****;**
**Figure 3** illustrates a sectional view of a portion of a sealed container according to embodiments of the present invention; and
**Figure 4** illustrates a metered dose inhaler according to embodiments of the present invention.

### Description of Preferred Embodiments of the Present Invention

The invention will now be described with respect to preferred embodiments described herein. It should be appreciated however that these embodiments are for the purpose of illustrating the invention, and are not to be construed as limiting the scope of the invention as defined by the claims. Like reference numerals refer to like elements throughout.

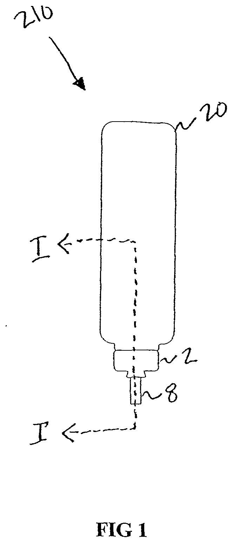

Referring first to **Figure 1**, a sealed container **210** according to embodiments of the present invention will be described. The sealed container **210** includes a container **20**. While the container **20** as illustrated in **Figure 1** is in the shape of a can or canister, it will be understood by those skilled in the art that the container **20** can have various other shapes including, but not limited to, spherical and oblong. The container **20** may be made of various materials as will be understood by those skilled in the art including, but not limited to, plastics, plastics-coated glass, and metal. The metal may be various metals as will be understood in the art including, but not limited to, aluminum and stainless steel. The metal is preferably aluminium or an alloy thereof which may optionally be anodised, lacquer-coated and/or plastic-coated (e.g., as described in U.S. Patent Nos. 6,131,566, 6,143,277, 6,149,892, 6,253,762, 6,511,652, 6,511,653, 6,524,555, 6,532,955, and 6,546,928 wherein part or all of the internal surfaces of the can are coated with one or more fluorocarbon polymers optionally in combination with one or more non-fluorocarbon polymers). The sealed container **210** is used to contain an aerosol pharmaceutical formulation, for example in a metered dose inhaler application, and is made of a material capable of withstanding the vapour pressure of the propellant used. Such materials include plastics, plastics-coated glass, and metal materials as described above.

The container **20** has an opening therein with a cap **2** covering the opening in the container **20**. A metering valve having a valve stem is positioned within the sealed container **210**. A portion **8** of the valve stem protrudes from the cap **2**. The cap **2** may be made of various materials as will be understood in the art including, but not limited to, plastic and metal. The cap is preferably made of a metal material such as stainless steel, aluminum or an aluminum alloy. The cap may be secured onto the canister via welding such as ultrasonic welding or laser welding, screw fitting or crimping. Preferably the container **20** is fitted with a cap assembly, wherein a metering valve is situated in the cap **2**, and the cap **2** is crimped in place.

In some embodiments, the medicament dispenser of the present invention includes the sealed container **210**.

In the present invention, the aerosol pharmaceutical formulation includes a combination of the medicaments salmeterol xinafoate and fluticasone propionate.

In some embodiments, the pharmaceutical formulation includes a combination of salmeterol xinafoate and fluticasone propionate and no further medicament substances are present.

The medicament is preferably present in the pharmaceutical formulation as a particulate medicament. The particle size of the particulate (e.g. micronised) medicament should be such as to permit inhalation of substantially all of the medicament into the lungs upon administration of the aerosol formulation and will thus be less than 100 microns, desirably less than 20 microns, and preferably in the range 1-10 microns, e.g. 1-5 microns.

The concentration of medicament in the formulation will generally be 0.01-10% such as 0.01-2%, particularly 0.01-1%, especially 0.03-0.25% w/w.

The aerosol pharmaceutical formulation in the present invention includes a propellant. The propellant is a hydrofluorocarbon propellant selected from the group consisting of 1,1,1,2-tetrafluoroethane (HFA 134a), 1,1,1,2,3,3,3-heptafluoro-n-propane (HFA 227) and mixtures thereof. In some embodiments, the propellant is a single propellant selected from HFA 134a and HFA 227. In other embodiments, the propellant is HFA 134a. It is desirable that the formulations are substantially free of chlorofluorocarbons such as CCl3F, CCl2F2 and CF3CCl3. Formulations which are substantially free of volatile adjuvants are preferred. In certain cases, it may be desirable to include appropriate amounts of water, which can be advantageous in modifying the dielectric properties of the propellant.

In some embodiments, it is desirable that the formulations of the invention are substantially free of polar adjuvants. "Substantially free" will generally be understood to mean containing less than 0.01% of polar adjuvant(s) especially less than 0.0001% based on weight of formulation.

It is preferable that the formulations of the invention are substantially free of surfactant.

The formulations for use in the invention may be prepared by dispersal of the medicament in the selected propellant in an appropriate container, for example, with the aid of sonication or a high-shear mixer. The process is desirably carried out under controlled humidity conditions.

The filling operation for filling a sealed container, such as the sealed container 20, with the aerosol pharmaceutical formulation may be performed utilizing conventional bulk manufacturing methods and machinery well known to those skilled in the art of pharmaceutical aerosol manufacture for the preparation of large scale batches for the commercial production of filled canisters. The particulate medicament is added to a charge vessel and liquefied propellant is pressure filled through the charge vessel into a manufacturing vessel, together with liquefied propellant containing the surfactant. The drug suspension is mixed before recirculation to a filling machine and an aliquot of the drug suspension is then filled through the metering valve into the sealed container.

In an alternative process, an aliquot of the liquefied formulation is added to an open container under conditions which are sufficiently cold such that the formulation does not vaporise, and then a metering valve crimped onto the canister.

Typically, in batches prepared for pharmaceutical use, each filled canister is check-weighed, coded with a batch number and packed into a tray for storage before release testing.

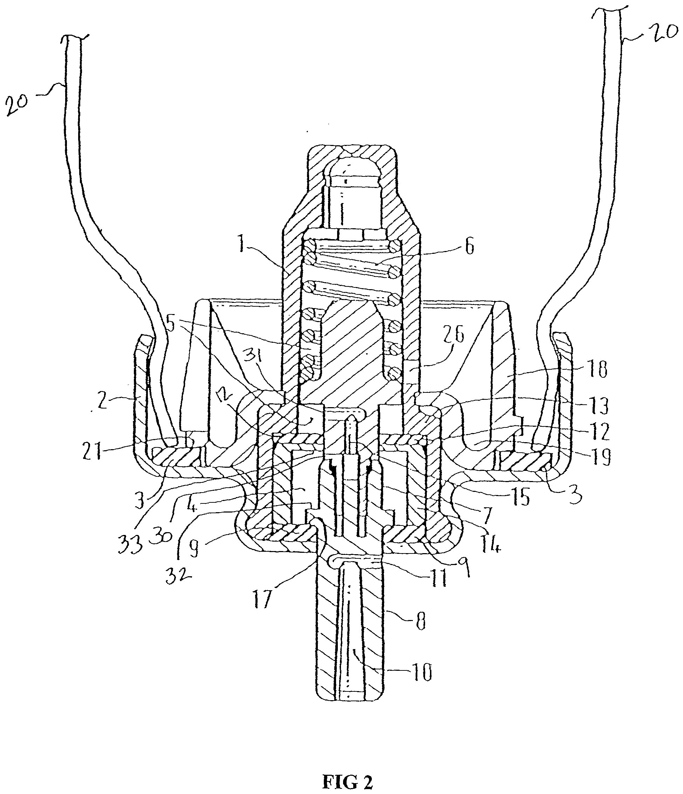

Referring now to **Figure 2**, a sectional view taken along line I-I illustrated in **Figure 1** of a lower portion of a sealed container according to the present invention will be described. A cap **2** covers the open end of a container **20**. A cap seal **3** is positioned between the open end of the container **20** and the cap **2**. As used herein, the term "seal" is used interchangeably with the terms "sealing gasket" or "gasket". A valve body **1** is positioned adjacent the cap **2**. The valve body **1** is formed such that its lower part defines a metering chamber **4**, and its upper part defines a sampling chamber **5**, which also acts as a housing for a return spring **6**. The words "upper" and ""lower" are used for the container when it is in a use orientation with the neck of the container **20** and valve at the lower end of the container which corresponds to the orientation of the valve as shown in **Figure 2**. The metering chamber preferably has a volume between 10 and 100 µl, more preferably between 20 and 80 µl. The valve body may comprise various materials as will be understood by those skilled in the art, including, but not limited to, plastic and metal materials. Inside the valve body is disposed a valve stem **7**, a part **8** of which extends outside the valve through lower stem seal **9** and cap **2**. The upper portion of the valve stem **7** has a diameter such that it can slide through an opening in an upper stem seal **12** and will engage the periphery of that opening sufficiently to provide a seal. The valve stem may comprise various materials as will be understood by those skilled in the art including, but not limited to, plastic and metal materials.

According to an embodiment of the present invention, the cap seal **3** comprises a first elastomeric material and the lower stem seal **9** and/or upper stem seal **12** comprise a second elastomeric material different from the first elastomeric material. The first elastomeric material comprises a polymer of ethylene propylene diene monomer (EPDM). The second elastomeric material comprises acrylonitrile butadiene rubbers.

In some embodiments of the present invention, the cap seal comprises the first elastomeric material and the upper stem seal and lower stem seal each comprise the second elastomeric material. In other words, in a preferred embodiment, the cap seal comprises an EPDM polymer and the upper stem seal and lower stem seal each comprise a nitrile polymer, such as acrylonitrile butadiene rubber.

Still referring to **Figure 2**, the upper stem seal **12** is held in position against a step **13** formed in the valve body **1** between the lower and upper parts by a sleeve **14** which defines the metering chamber **4** between the lower stem seal **9** and upper stem seal **12**. The stem part **8** is formed with an inner axial or longitudinal canal **10** opening at the outer end of the stem and in communication with a radial passage **11**. The valve stem **7** has a passage **15** which, when the stem is in the inoperative position shown, provides fluid communication between the metering chamber **4** and sampling chamber **5** via orifices **30** and **31**, respectively. The sampling chamber **5** is in fluid communication with the interior of the container **20** via orifice **26** formed in the side of the valve body.

The valve stem **7** is biased downwardly to the inoperative position by the return spring **6** and is provided with a shoulder **17** which abuts against the lower stem seal **9**. In the inoperative position as shown in **Figure 2**, the shoulder **17** abuts against the lower stem seal **9** and the radial passage **11** opens below the lower stem seal **9** so that the metering chamber **4** is isolated from the canal **10** and the pharmaceutical formulation contained within the container **20** cannot escape.

A ring **18** having a "U" shaped cross section extending in a radial direction is disposed around the valve body below orifice **26** so as to form a trough **19** around the valve body. As seen in **Figure 2**, the ring is formed as a separate component having an inner annular contacting rim of a diameter suitable to provide a friction fit over the upper part of valve body **1**. The ring seats against step **13** below the orifice **26**. While the ring **18** is illustrated in **Figure 2** as being separate from the valve body **1**, it will be understood by those skilled in the art that the ring **18** may alternatively be formed as an integrally molded part of valve body **1**. In some embodiments, the valve stem, the valve body, and/or at least a portion of the metering chamber wall(s) present a surface to the pharmaceutical formulation to which the one or more medicaments in the pharmaceutical formulation are non-adherent (e.g., as described in WO99/42154, WO97/16360, and WO99/50156). For example, the metering chamber (especially when composed of a plastics material) may be surface treated so as to present a substantially fluorinated surface to the formulation. Alternatively the metering chamber (especially when composed of a plastics material) may be surface treated with a siloxane such as dimethyl siloxane. As a further alternative, the metering chamber presents a substantially fluorinated surface to the formulation by virtue of being composed of a suitable substantially fluorinated material. Suitable metering chambers and surface treatments for metering chambers are described in WO 02/51483 at page 7, line 15 to page 11, line 18, for example. Suitable valve stems and surface treatments for valve stems are described in WO 02/51483 at page 11, line 21 to page 12, line 3, for example.

To use the device illustrated in **Figure 2**, the sealed container is first shaken to homogenise the suspension within the container **20**. The user then depresses the valve stem **7** against the force of the spring **6**. When the valve stem is depressed, the shoulder **32** on the valve stem **7** comes to rest on a surface **33** of the sleeve **14**. The orifice **30** comes to lie on the side of the upper stem seal **12** remote from the metering chamber **4**, thereby isolating the metering chamber **4** from the sampling chamber **5**. The radial passage **11** is moved into the metering chamber **4**, creating fluid communication between the metering chamber **4** and the outlet canal **10** in the valve stem **7**. Thus, the metered dose being held in the metering chamber **4** can exit through the radial passage **11** and the outlet canal **10**.

Releasing the valve stem **7** causes it to return to the illustrated position under the force of the spring **6**. The passage **15** then once again provides fluid communication between the metering chamber **4** and the sampling chamber **5**. Accordingly, at this stage, liquid pharmaceutical formulation passes under pressure from the container **20** through orifice **26**, through orifice **31**, through passage **15**, through orifice **30**, and into the metering chamber **4** to fill the metering chamber **4**.

Metering valves for use in the present invention may be made by various methods as will be understood by those skilled in the art.

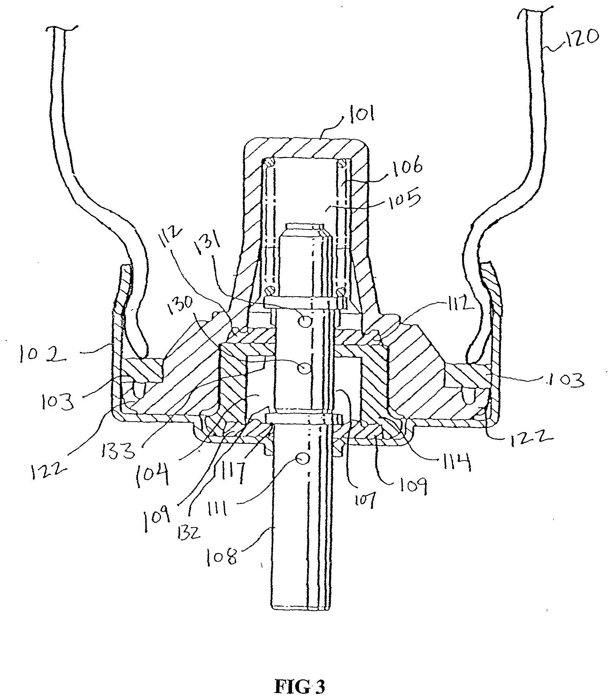

Referring now to **Figure 3**, a sectional view of a lower portion of a sealed container according to the present invention will be described. The elements referred to by reference numerals **102, 103, 104, 105, 107, 108, 109, 111, 112, 114, 117, 120, 130, 131, 132,** and **133** are similar to the elements referred to by reference numerals **2, 3, 4, 5, 7, 8, 9, 11, 12, 14, 17, 20, 30, 31, 32,** and **33** described above in **Figure 2** and will not be further described. A valve body **101** is formed such that its lower part defines the metering chamber **104**, its upper part defines the sampling chamber **105**, which also acts as a housing for a resilient member **106**, and a portion of the valve body **122** that supports the cap seal **103**. The valve body may comprise various materials such as those described above with reference to the valve body **1** in **Figure 2**. The resilient member **106** is used to bias the valve stem **107** towards the upper surface of the lower stem seal **109**. The resilient member **106** may comprise various resilient members as will be understood by those skilled in the art including, but not limited to, a spring, and a flexible bushing.

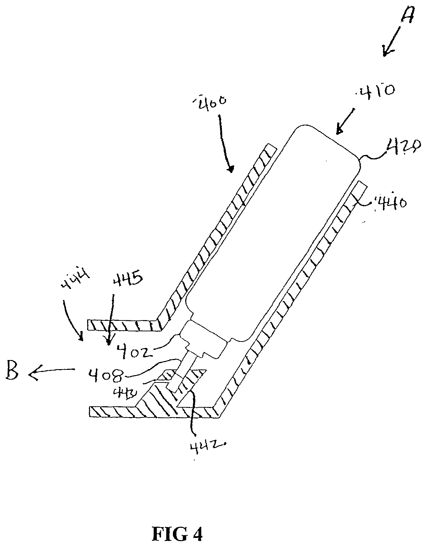

Referring now to **Figure 4**, a metered dose inhaler **400** according to embodiments of the present invention will be described. The metered dose inhaler **400** includes a medicament dispenser comprising a sealed container **410** that is fitted within an actuator housing **440**. The sealed container **410** includes a container **420** having an opening therein with a cap **402** covering the opening in the container **420.** A metering valve having a valve stem **408** is positioned within the sealed container **410**. The valve stem **408** is engaged with a nozzle block **442**, which is integrally formed with the actuator housing **440**. While the nozzle block **442** is illustrated in **Figure 4** as being integrally formed with the actuator housing **440**, it will be understood by those skilled in the art that the nozzle block may be formed separately from the actuator housing. The actuator housing **440** is illustrated as an oral inhalation actuator housing. Metered dose inhalers according to embodiments of the present invention are designed to deliver a fixed unit dosage of medicament per actuation or "puff", for example, in the range of 2.5 to 5000 micrograms of medicament per puff, preferably in the range of from 5.0 to 2500 micrograms per puff.

MDIs taught herein may be prepared by various methods as will be understood by those skilled in the art (e.g., see Byron, above and WO/96/32150).

Referring to **Figure** 4, a metered dose of the pharmaceutical formulation may be administered from the metered dose inhaler **400** by the patient placing his/her mouth over the opening in the actuator **444** and pressing the sealed container **410** into the actuator housing **440** along direction **A** while inhaling. Pressing the sealed container **410** into the actuator housing **440** will cause the end of the valve stem **408** to engage the nozzle block, thus actuating the metering valve in the sealed container **410**. A metered dose of the pharmaceutical formulation will then exit the nozzle block via orifice **443**, exit the actuator via a cylindrical or cone-like passage **445** through which medicament may be delivered from the filled canister via the metering valve to the mouth of the patient along direction **B** and be drawn into the patient's lungs.

Administration of medicament in a container or MDI in accordance with embodiments of the present invention may be indicated for the treatment of mild, moderate, severe acute or chronic symptoms or for prophylactic treatment. It will be appreciated that the precise dose administered will depend on the age and condition of the patient, the particular particulate medicament used and the frequency of administration and will ultimately be at the discretion of the attendant physician. When combinations of medicaments are employed the dose of each component of the combination will in general be that employed for each component when used alone.

Suitable daily doses, may be, for example, in the range 50 to 200 micrograms of salmeterol or 50 to 2000 micrograms of fluticasone propionate, depending on the severity of the disease. Thus, for example, each valve actuation may deliver 25 micrograms of salmeterol or 25, 50, 125 or 250 micrograms of fluticasone propionate. Typically each filled canister for use in a metered dose inhaler contains 60, 100, 120, 160 or 240 metered doses or puffs of medicament.

According to still other embodiments of the present invention, a packaging material forming an enclosed volume contains the metered dose inhaler.

The packaging material may be various packaging material as will be understood by those skilled in the art including, but not limited to, cartons and flexible wrappers. In some embodiments, the packaging material is a flexible wrapper that comprises a material that is substantially impermeable to ingress of atmospheric moisture and, optionally, substantially permeable to egress of propellant gas (e.g., as described in U.S. Patent Nos. 6,119,853, 6,179,118, 6,315,112, 6,352,152, and 6,390,291). Preferably, the package will also contain within it a desiccant material as will be understood by those skilled in the art. The desiccant material may be inside the MDI and/or outside the MDI.

The packaging operation may be performed by various processes as will be understood by those skilled in the art, including but not limited to, those described in U.S. Patent Nos. 6,119,853, 6,179,118, 6,315,112, 6,352,152, and 6,390,291.

It has been found that the absolute FPM measurements (before or after storage) are higher in a medicament dispenser (and/or an MDI) according to embodiments of the present invention than in a conventional medicament dispenser (and/or an MDI), which utilizes the same elastomeric material for the can seal and the one or more stem seals. Without being bound by any particular theory, it is, at the time of filing, hypothesised that embodiments of the present invention provide advantageous stabilisation of the aerosol formulation by one or more of the following effects: reducing drug deposition, improving stability of FPM even after storage, decreasing the increase in mean mass aerodynamic diameter (MMAD) during storage, and/or decreasing the GSD (Geometric Standard Deviation).

In embodiments of the invention, there is provided a metered dose inhaler having a shelf-life that is longer than the shelf-life of a conventional metered dose inhaler that includes a cap seal and a stem seal that comprises the same elastomeric material. In some embodiments, the shelf-life is measured by determining the FPM of the pharmaceutical formulation after storage under conditions such as 25, 30 or 40°C and 50, 60, 75, or 85% relative humidity (RH) (preferred conditions are 25°C/60% RH, 25°C/75% RH, 30°C/50% RH, 30°C/60% RH, 40°C/75% RH, or 40°C/85% RH) for a time period such as 1, 4, 12, 26, or 52 weeks and comparing the determined FPM to the initial FPM. In these embodiments, the shelf life will be longer if, at the same or similar storage conditions, it takes a longer time period before the determined FPM reaches a given level. For example, if a conventional MDI exhibits a drop in FPM of 20% after storage at 20°C/75% RH for 4 weeks and an MDI of the present invention exhibits a drop in FPM of 20% after storage at 20°C/75% RH for 26 weeks, the MDI of the present invention will have a prolonged shelf-life. In some embodiments, the shelf-life is prolonged by at least 1, 2, 4, 8, or 12 weeks.

According to other embodiments of the present invention, the FPM of the particulate medicament is maintained within 15%, more preferably within 10% and especially within 5% of its original level after 4, 8, and preferably 12 weeks storage at 40°C and 75% relative humidity.

The chemical and physical stability and the pharmaceutical acceptability of the aerosol formulations according to the invention may be determined by techniques well known to those skilled in the art. Thus the chemical stability of the components may be determined by HPLC assay, for example, after prolonged storage of the product. Physical stability data may be gained from other conventional analytical techniques such as by leak testing, by valve delivery assay (average shot weights per actuation), by dose reproducibility assay (active ingredient per actuation) and spray distribution analysis.

The suspension stability of the aerosol formulations according to the invention may be measured by conventional techniques, for example, by measuring flocculation size distribution using a back light scattering instrument or by measuring aerodynamic particle size distribution by cascade impaction, next generation impactor, multistage liquid impinger, or by the "twin impinger" analytical process. As used herein reference to the "twin impinger" assay means "Determination of the deposition of the emitted dose in pressurised inhalations using apparatus A" as defined in British Pharmacopaeia 1988, pages A204-207, Appendix XVII C. Such techniques enable the "respirable fraction" of the aerosol formulations to be calculated. One method used to calculate the "respirable fraction" is by reference to "fine particle fraction" which is the amount of active ingredient collected in the lower impingement chamber per actuation expressed as a percentage of the total amount of active ingredient delivered per actuation using the twin impinger method described above. As discussed above, the absolute "fine particle mass" (FPM) is an important parameter in relation to the present invention. The FPM may be assessed using the same apparatus as the fine particle fraction.

Except as otherwise noted, all references including patent and published patent applications referred to herein are incorporated herein by reference in their entireties.

The invention will now be described further with reference the following Examples which serve to illustrate the invention but are not intended to be limiting.

### Examples

### Example I

Sealed containers including an 8 ml aluminium canister (manufactured by Presspart Inc., of Cary, North Carolina) coated with a PTFE-PES coating supplied by CCL Container of Harrisonburg, Virginia, a neck (or cap) seal, a cap (or ferule) and a DF60 Mk42 metering valve, item no. 803309, (manufactured by Valois Pharm, of Le Vaudreuil, France) having a lower stem seal and an upper stem seal were assembled using conventional techniques known in the art. The materials used for the neck seal, the lower stem seal, and the upper stem seal in each of the sealed containers were varied according to the following matrix.

*** For comparative purposes only. Not part of the present invention.**

| Sealed container | Neck Seal | Lower Stem Seal | Upper Stem Seal |
|---|---|---|---|
| 1* | EPDM | EPDM | EPDM |
| 2 | EPDM | EPDM | Nitrile |
| 3 | EPDM | Nitrile | EPDM |
| 4 | EPDM | Nitrile | Nitrile |
| 5 | Nitrile | EPDM | EPDM |
| 6 | Nitrile | EPDM | Nitrile |
| 7 | Nitrile | Nitrile | EPDM |
| 8* | Nitrile | Nitrile | Nitrile |

The EPDM seals were model no. 808TS1 and/or 808TS1 EX2 seals obtained from Valois Pharm and had been extracted with ethanol. The nitrile seals were acrylonitrile butadiene rubber seals, model no. 403B and/or 404B, obtained from Valois Pharm.

The sealed containers were then filled through the metering valve with a pharmaceutical formulation containing 8 mg fluticasone propionate and 5.8 mg salmeterol xinafoate in 12 grams of 134a propellant. After filling, the sealed containers were fired and the initial fine particle mass (FPM) of the formulation was determined for each container using Anderson Cascade Impaction, with the FPM being the sum of the 3, 4, and 5 stage values.

After determining the initial FPM, the sealed containers were stored at 40°C/75% RH for 12 weeks. The FPM was then determined again using the procedure described above. The relative FPM results (with variability) are illustrated in Chart 1 below:

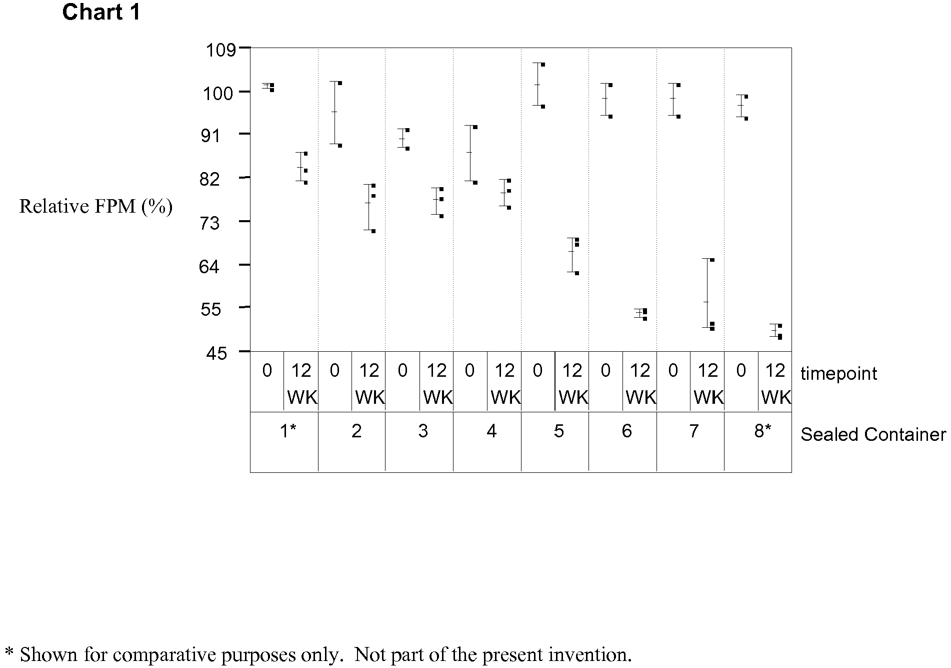

As can be seen in Chart 1, sealed containers 2, 3, and 4 having a neck seal made of EPDM and at least one stem seal made of nitrile exhibited improved stability (e.g., lower drops in FPM after storage) when compared to the conventional sealed container 8 having all nitrile seals.

### Example II

The procedures performed in Example I above were repeated using a pharmaceutical formulation similar to that used in Example I and using sealed containers similar to those used in Example I, with the exception that the valves were DF60 Mk42 metering valves, item no. 10002715, (manufactured by Valois Pharm, of Le Vaudreuil, France). The relative FPM results (with variablility) are illustrated in Chart 2 below:

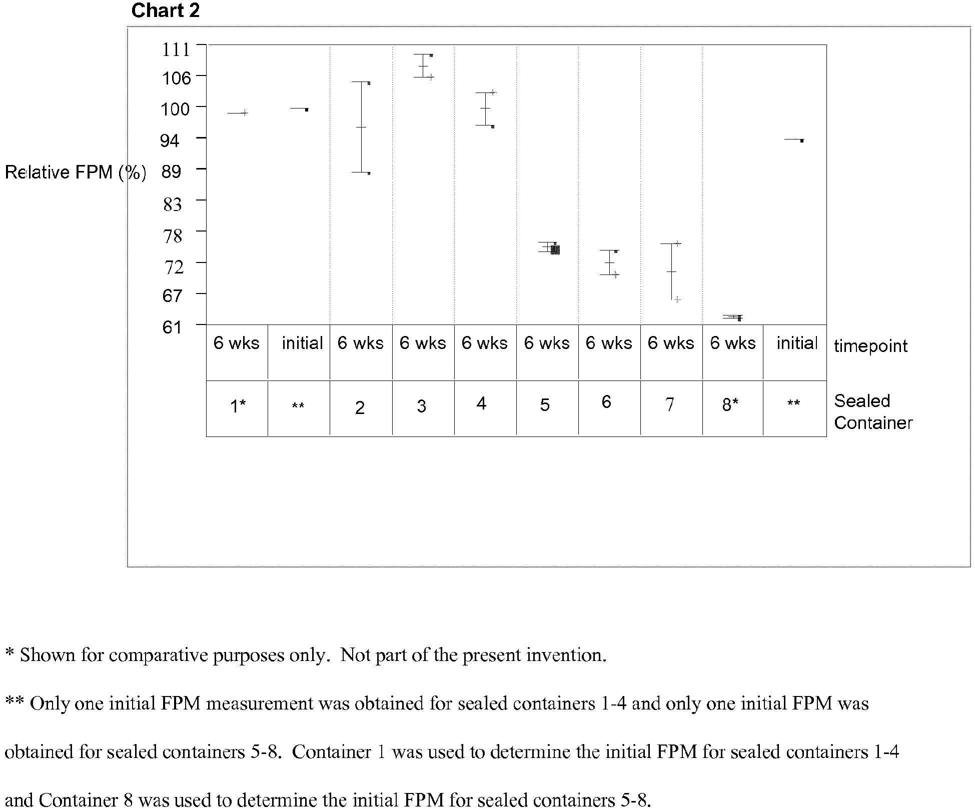

As can be seen in Chart 2, sealed containers 2, 3, and 4 having a neck seal made of EPDM and at least one stem seal made of nitrile exhibited improved stability (e.g., lower or no measurable drop in FPM after storage) when compared to the conventional sealed container 8 having all nitrile seals.

## Claims

1. A medicament dispenser comprising:
a sealed container (210;410) that comprises:
a container (20;120;420) having an opening therein;
a cap (2;102;402) covering the opening in the container;
a metering valve (1 ;101) adjacent the cap, said metering valve comprising at least one stem seal (9,12;109,112); and
a cap seal (3;103) positioned between the cap and the container to provide a sealed container to contain an aerosol pharmaceutical formulation,
**characterised by:**
said at least one stem seal comprising a nitrile polymer;
said cap seal comprising an ethylene propylene diene monomer (EPDM) polymer; and
an aerosol pharmaceutical formulation contained within the sealed container comprising salmeterol xinafoate, fluticasone propionate and a propellant selected from the group consisting of 1,1,1,2-tetrafluoroethane (HFA 134a), 1,1,1,2,3,3,3-heptafluoro-n-propane (HFA 227) and mixtures thereof.

2. The medicament dispenser of claim 1, wherein the metering valve comprises a first stem seal (9; 109) and a second stem seal (12; 112) and wherein at least one of said first and second stem seals comprises nitrile polymer.

3. The medicament dispenser of claim 2, wherein the first stem seal and the second stem seal each comprise nitrile polymer.

4. The medicament dispenser of claim 3, wherein the first stem seal and the second stem seal each have similar extractant profiles.

5. A metered dose inhaler (400) comprising:
the medicament dispenser according to any one of claims 1 to 4; and
an actuator (440) engaging the medicament dispenser and configured to dispense the pharmaceutical formulation from the medicament dispenser.

## Patentansprüche

1. Arzneimittelspender, umfassend:
einen verschlossenen Behälter (210;410), der umfasst:
einen Behälter (20;120;420) mit einer Öffnung darin;
einen Verschluss (2;102;402), der die Öffnung in dem Behälter abdeckt;
ein Dosierventil (1;101) benachbart zu dem Verschluss, wobei das Dosierventil mindestens eine Schaftdichtung (stem seal) (9,12;109,112) umfasst; und
eine Verschlussdichtung (3;103), die so zwischen dem Verschluss und dem Behälter angeordnet ist, dass sie einen verschlossenen Behälter bereitstellt, der eine pharmazeutische Aerosolformulierung enthalten soll,
**dadurch gekennzeichnet, dass**:
die mindestens eine Schaftdichtung ein Nitrilpolymer umfasst;
die Verschlussdichtung ein Ethylen-Propylen-Dienmonomer (EPDM)-Polymer umfasst; und
eine pharmazeutische Aerosolformulierung, die in dem verschlossenen Behälter enthalten ist, Salmeterolxinafoat, Fluticasonpropionat und ein Treibmittel, ausgewählt aus der Gruppe bestehend aus 1,1,1,2-Tetrafluorethan (HFA 134a), 1,1,1,2,3,3,3-Heptafluor-n-propan (HFA 227) und Mischungen davon, umfasst.

2. Arzneimittelspender nach Anspruch 1, wobei das Dosierventil eine erste Schaftdichtung (9;109) und eine zweite Schaftdichtung (12;112) umfasst und wobei mindestens eine der ersten und zweiten Schaftdichtungen Nitrilpolymer umfasst.

3. Arzneimittelspender nach Anspruch 2, wobei die erste Schaftdichtung und die zweite Schaftdichtung jeweils Nitrilpolymer umfassen.

4. Arzneimittelspender nach Anspruch 3, wobei die erste Schaftdichtung und die zweite Schaftdichtung jeweils ähnliche Extraktionsprofile aufweisen.

5. Dosierinhalator (400), umfassend:
den Arzneimittelspender gemäß mindestens einem der Ansprüche 1 bis 4; und
einer Betätigungsvorrichtung (actuator) (440), in die der Arzneimittelspender eingreift und so konfiguriert ist, dass sie die pharmazeutische Formulierung aus dem Medikamentenspender abgibt.

## Revendications

1. Distributeur de médicament comprenant :
un récipient étanchéifié (210 ; 410) qui comprend :
un récipient (20 ; 120 ; 420) renfermant une ouverture ;
un capuchon (2 ; 102 ; 402) couvrant l'ouverture dans le récipient ;
une valve doseuse (1 ; 101) adjacente au capuchon, ladite valve doseuse comprenant au moins un joint de tige (9, 12 ; 109, 112) ; et
un joint de capuchon (3 ; 103) positionné entre le capuchon et le récipient pour fournir un récipient étanchéifié en vue de contenir une formulation pharmaceutique aérosol,
**caractérisé en ce que** :
ledit au moins un joint de tige comprend un polymère de nitrile ;
ledit joint de capuchon comprend un polymère de monomère d'éthylène-propylène-diène (EPDM) ; et
une formulation pharmaceutique aérosol contenue dans le récipient étanchéifié comprend du xinafoate de salmétérol, du propionate de fluticasone et un gaz propulseur choisi dans le groupe consistant en le 1,1,1,2-tétrafluoroéthane (HFA 134a), 1,1,1,2,3,3,3,-heptafluoro-n-propane (HFA 227) et leurs mélanges.

2. Distributeur de médicament selon la revendication 1, dans lequel la valve doseuse comprend un premier joint de tige (9 ;109) et un second joint de tige (12 ; 112) et dans lequel au moins l'un desdits premier et second joints de tige comprend un polymère de nitrile.

3. Distributeur de médicament selon la revendication 2, dans lequel ledit premier joint de tige et ledit second joint de tige comprennent chacun un polymère de nitrile.

4. Distributeur de médicament selon la revendication 3, dans lequel le premier joint de tige et le second joint de tige ont chacun des profils d'extractant similaires.

5. Inhalateur-doseur (400) comprenant :
le distributeur de médicament selon l'une quelconque des revendications 1 à 4 ; et
un actionneur (440) enclenchant le distributeur de médicament et configuré pour distribuer la formulation pharmaceutique à partir du distributeur de médicament.
